# EUROPÄISCHE PATENTSCHRIFT

(11) **EP 3 004 042 B1**
(45) Veröffentlichungstag und Bekanntmachung des Hinweises auf die Patenterteilung: **24.10.2018**
(21) Anmeldenummer: 14725438.7
(22) Anmeldetag: 20.05.2014
(51) Int. Cl.: C07C 45/45, C07C 45/62, C07C 49/798

(54) **PHOTOLABILE DUFTSPEICHERSTOFFE**
PHOTOLABILE PRO-FRAGRANCES
INGRÉDIENTS ODORIFÉRANTS PHOTOLABILES

(30) Priorität: 29.05.2013 DE 102013209988
(43) Veröffentlichungstag der Anmeldung: 13.04.2016
(73) Patentinhaber: Henkel AG & Co. KGaA, 40589 Düsseldorf (DE)
(72) Erfinder: GERKE, Thomas, 40627 Düsseldorf (DE); KROPF, Christian, 40724 Hilden (DE); HUCHEL, Ursula, 50733 Köln (DE); GRIESBECK, Axel, 50937 Köln (DE); PORSCHEN, Björn, 51069 Köln (DE)
(74) Vertreter: Viering, Jentschura & Partner mbB Patent- und Rechtsanwälte
(86) Internationale Anmeldenummer: PCT/EP2014/060331
(87) Internationale Veröffentlichungsnummer: WO 2014/191256

(56) Entgegenhaltungen:
- WO-A1-2011/101179
- US-A1- 2010 137 178
- KILSUNG LEE ET AL: "One-pot synthesis of alpha, betha-unsaturated ketones", SYNTHESIS, 1. Januar 1991 (1991-01-01), Seiten 213-214, XP055146870,

## Beschreibung

Die vorliegende Erfindung betrifft ein Verfahren zur Darstellung von Duftspeicherstoffen, ausgewählte Duftspeicherstoffe selbst und Mittel, in denen solche Duftspeicherstoffe eingesetzt werden.

Wasch- oder Reinigungsmittel, kosmetische Mittel, Klebstoffe oder Druckfarben enthalten zumeist Duftstoffe, die den Mitteln einen angenehmen Geruch verleihen. Die Duftstoffe maskieren dabei nicht nur einen eventuellen Geruch der anderen Inhaltstoffe, sondern lassen beim Verbraucher auch einen angenehmen Geruchseindruck entstehen.

Insbesondere im Bereich der Wasch- und Reinigungsmittel sind Duftstoffe wichtige Bestandteile der Zusammensetzung, da die Wäsche sowohl im feuchten als auch im trockenen Zustand einen angenehmen und möglichst auch frischen Duft aufweisen soll.

Grundsätzlich steht man bei dem Einsatz von Duftstoffen in Wasch- oder Reinigungsmitteln, kosmetischen Mitteln, Klebstoffen oder Druckfarben vor dem Problem, dass es sich bei den Duftstoffen um mehr oder minder leicht flüchtige Verbindungen handelt, aber dennoch ein langanhaltender Dufteffekt angestrebt wird. Insbesondere bei denjenigen Duftstoffen, die die frischen und leichten Noten des Parfüms darstellen und infolge ihres hohen Dampfdrucks besonders schnell abdampfen, ist die gewünschte Langlebigkeit des Dufteindrucks kaum erreichbar.

Eine Möglichkeit zur verzögerten Freisetzung von Duftstoffen stellt der Einsatz von sogenannten photoaktivierbaren Substanzen als Duftspeicherstoffe dar. Durch die Einwirkung des Sonnenlichts oder einer anderen elektromagnetischen Strahlung bestimmter Wellenlänge wird der Bruch einer kovalenten Bindung im Duftspeicherstoff-Molekül induziert, wodurch ein Duftstoff freigesetzt wird.

In diesem Zusammenhang offenbart die WO 2011/101179 A1 spezielle Ketone als photoaktivierbare Substanzen, die in Gegenwart von Sonnenlicht in einer photochemischen Fragmentierung einen Duftstoff mit mindestens einer cyclischen Doppelbindung freisetzen.

Die genannten Duftspeicherstoffe werden dabei ausgehend vom Duftstoff in einer zweistufigen Synthese über eine Hydroborierung der mindestens einen cyclischen Doppelbindung und einer anschließenden Substitutionsreaktion dargestellt.

Ein Nachteil des in der WO 2011/101179 A1 beschriebenen Syntheseverfahrens ist, dass Duftstoffe, die neben der mindestens einen cyclischen Doppelbindung zusätzlich eine oder mehrere semicyclische und/oder exocyclische Doppelbindungen besitzen, nicht selektiv oder gar nicht zu den gewünschten Duftspeicherstoffen umgesetzt werden können, da während der Hydroborierung des Duftstoffs regioselektiv die sterisch am wenigsten gehinderte Doppelbindung zuerst reagiert. Ein Nachteil dieser unerwünschten Konstitutionsisomere ist, dass sie bei Bestrahlung mit Licht nur unzureichend oder gar nicht wieder gespalten werden und den gespeicherten Duftstoff nicht wieder freigeben.

Aufgabe der vorliegenden Erfindung war deshalb die Bereitstellung eines einfachen Verfahrens zur selektiven Darstellung eines Duftspeicherstoffs, der einerseits eine effektive Freisetzung des gespeicherten Duftstoffs gewährleistet und der andererseits Duftstoffe speichert, die neben mindestens einer cyclischen Doppelbindung zusätzliche semicyclische und/oder exocyclische Doppelbindungen aufweisen können.

Gelöst wurde diese Aufgabe durch ein Verfahren zur Herstellung eines Ketons der allgemeinen Formel (I) 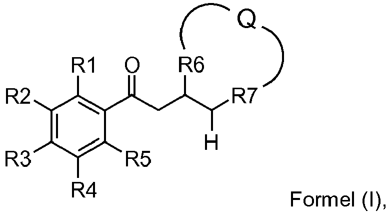
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können und
R6 und R7 unabhängig voneinander für ein sekundäres, tertiäres oder quartäres C-Atom stehen und
Q für eine R6 und R7 verbrückende substituierte oder unsubstituierte Gruppe mit 1 bis 10 Kohlenstoffatomen steht, bei dem
   a) ein Keton der allgemeinen Formel (II) 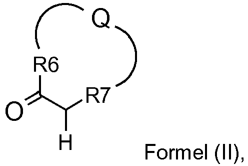 wobei die Reste R6, R7 und Q identisch sind zur allgemeinen Formel (I),
      in Gegenwart eines Phosphonats der allgemeinen Formel (III) 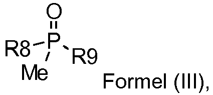 wobei R8 und R9 jeweils unabhängig voneinander für eine lineare oder verzweigte, substituierte oder unsubstituierte Alkoxygruppe mit 1 bis 15 Kohlenstoffatomen stehen,
      mit einem Benzonitril der allgemeinen Formel (IV) 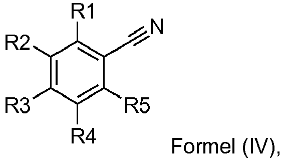 wobei die Reste R1, R2, R3, R4 und R5 identisch sind zur allgemeinen Formel (I), umgesetzt wird und anschließend
   b) das unter Schritt a) erhaltene alpha,beta-ungesättigte Keton der allgemeinen Formel (V) 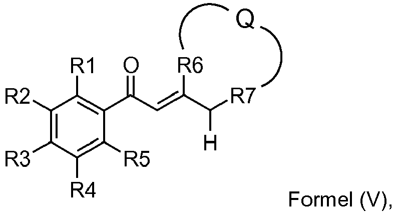 wobei die Reste R1 bis R7 und Q identisch sind zur allgemeinen Formel (I),
      selektiv zu einem Keton der allgemeinen Formel (I) hydriert wird.

Unter dem Begriff sekundäres C-Atom ist im Sinne der Erfindung ein Kohlenstoffatom zu verstehen, dass mit zwei weiteren Kohlenstoffatomen kovalent verbunden ist. Unter dem Begriff tertiäres C-Atom bzw. quartäres C-Atom ist im Sinne der Erfindung ein C-Atom zu verstehen, dass mit drei bzw. vier weiteren Kohlenstoffatomen kovalent verbunden ist, wobei ein tertiäres C-Atom bzw. ein quartäres C-Atom im Sinne der Erfindung auch ein Kohlenstoffatom sein kann, das mit nur zwei (tertiär) bzw. drei (quartär) weiteren Kohlenstoffatomen kovalent verbunden ist und zu einem der beiden bzw. drei Kohlenstoffatome eine Doppelbindung ausbildet.

Es konnte überraschend gefunden werden, dass durch das erfindungsgemäße Verfahren auf einfache Art und Weise selektiv Duftstoffspeicher der allgemeinen Formel (I) erhalten werden können, in denen Duftstoffe mit mindestens einer cyclischen Doppelbindung gespeichert sind und die durch Bestrahlung mit Licht den gespeicherten Duftstoff mit mindestens einer cyclischen Doppelbindung effektiv wieder freisetzen.

Bei dem Begriff "Duftstoff" handelt es sich im Sinne dieser Erfindung nicht um das Keton der allgemeinen Formel (II) zur Synthese des Duftspeicherstoffs der allgemeinen Formel (I), sondern um die Verbindung, die bei Belichtung aus dem Duftspeicherstoff freigesetzt wird. Dies unterscheidet das erfindungsgemäße Verfahren von dem in der WO 2011/101179 A1 beschriebenen Verfahren, wo der freizusetzende Duftstoff als Ausgangsverbindung eingesetzt wird.

In einer bevorzugten Ausführungsform der Erfindung weist das Keton der allgemeinen Formel (II) mindestens eine semicyclische oder exocyclische Doppelbindung auf.

Duftstoffe mit mindestens einer cyclischen Doppelbindung und mindestens einer semicyclischen oder exocyclischen Doppelbindung konnten bisher nicht selektiv in Duftspeicherstoffen der allgemeinen Formel (I) gespeichert werden, da der Hydroborierungsschritt des im Stand der Technik bekannten Verfahrens zur Darstellung solcher Duftspeicherstoffe bevorzugt an der sterisch am wenigsten gehinderten Doppelbindung erfolgt. Das erfindungsgemäße Verfahren dagegen ermöglicht unabhängig von der Anzahl semicyclischer oder exocyclischer Doppelbindungen des freigesetzten Duftstoffs die selektive Darstellung des gewünschten Duftspeicherstoffs der allgemeinen Formel (I).

In einer weiteren bevorzugten Ausführungsform der Erfindung handelt es sich bei dem verbrückenden Teil -CH-R7-Q-R6- des Ketons der allgemeinen Formel (II) um einen Kohlenwasserstoff.

Die Duftstoffe zu dem oben beschriebenen Keton der allgemeinen Formel (II), bei denen die Carbonylgruppe des Ketons der allgemeinen Formel (II) eine Methylen- oder Methingruppe ist, zeichnen sich durch ihren hohen Dampfdruck aus und sind aufgrund ihres oft niedrigen Funktionalisierungsgrads chemisch nur schwer an herkömmliche Trägermedien zu binden. Durch das erfindungsgemäße Verfahren ist es nun möglich Duftspeicherstoffe bereitzustellen, die diese Duftstoffe über einen längeren Zeitraum hinweg gezielt freisetzen.

In einer besonders bevorzugten Ausführungsform der Erfindung handelt es sich bei dem Keton der allgemeinen Formel (II) um Dihydrocarvon.

Aus Dihydrocarvon wird nach dem erfindungsgemäßen Verfahren ein Duftspeicherstoff der allgemeinen Formel (I) erhalten, der bei der Bestrahlung mit Licht Limonen freisetzt, welches einer der wichtigsten Duftstoffe auf dem Gebiet der Wasch- und Reinigungsmittel ist. Der Geruch von Limonen wird häufig mit Frische verbunden, was beim Verbraucher als Synonym für Sauberkeit/Reinheit steht.

In einer weiteren bevorzugten Ausführungsform der Erfindung sind die Reste R8 und R9 des Phosphonats der allgemeinen Formel (III) jeweils unabhängig voneinander Methoxy-, Ethoxy-, Propoxy-, Butoxy- oder Isopropoxy-Reste.

Phosphonate mit diesen Resten R8 und R9 bieten einen guten Kompromiss aus guter Löslichkeit und Stabilität des Phosphonats, geringem sterischen Anspruch und guter Reaktivität.

In einer weiteren bevorzugten Ausführungsform der Erfindung stehen die Reste R1 bis R5 jeweils unabhängig voneinander für Wasserstoff, Methyl- oder Methoxygruppen, wobei Methoxygruppen besonders bevorzugt sind.

Nitrile mit diesen Resten R1 bis R5 sind entweder kommerziell verfügbar oder synthetisch gut zugänglich und beeinflussen das Absorptionsspektrum des Duftspeicherstoffs auf vorteilhafte Weise. So kann durch geeignete Wahl der Reste R1 bis R5 die Freisetzungsgeschwindigkeit des gespeicherten Duftstoffs bei Bestrahlung mit vorzugsweise natürlichem Sonnenlicht oder handelsüblichen Leuchtmitteln erhöht oder verlangsamt werden.

In noch einer weiteren bevorzugten Ausführungsform der Erfindung ist das Hydriermittel ausgewählt aus der Gruppe der Alkalimetallborhydride, wobei Natriumborhydrid besonders bevorzugt ist.

Alkalimetallborhydride und insbesondere Natriumborhydrid sind aufgrund ihrer festen Form einfach handhabbar und hydrieren selektiv die semicyclische Doppelbindung des alpha,beta-ungesättigten Ketons (V).

Ein weiterer Gegenstand der Erfindung ist ein Keton der allgemeinen Formel (VI), 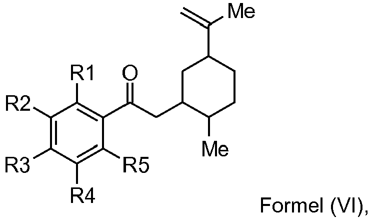 wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können, herstellbar durch
a) die Umsetzung von Dihydrocarvon mit einem Benzonitril der allgemeinen Formel (IV) in Gegenwart eines Phosphonats der allgemeinen Formel (III), und anschließender
b) selektiver Hydrierung des in Schritt a) erhaltenen alpha,beta-ungesättigten Ketons zu einem Keton der allgemeinen Formel (VI).

Ein Keton der allgemeinen Formel (VI) ist aus parfümistischer Sicht besonders interessant, da der gespeicherte Duftstoff (Limonen) eine der wichtigsten Basiskomponenten vieler Parfümzusammensetzungen ist. Freies Limonen besteht ausschließlich aus Kohlenstoff und Wasserstoff, ist leicht flüchtig und besitzt neben seiner cyclischen Doppelbindung eine exocyclische Doppelbindung, weshalb die gezielte Speicherung von Limonen unter Anwendung des Verfahrens gemäß WO 2011/101179 A1 als Keton der allgemeinen Formel (VI) bisher nicht möglich war.

Ein weiterer Gegenstand der Erfindung ist ein Mittel enthaltend ein Keton der allgemeinen Formel (I) 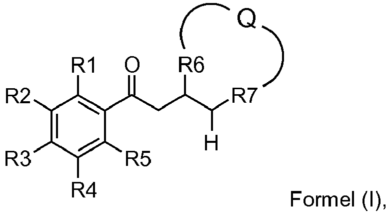
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können und
R6 und R7 unabhängig voneinander für ein sekundäres, tertiäres oder quartäres C-Atom stehen und
Q für eine R6 und R7 verbrückende substituierte oder unsubstituierte Gruppe mit 1 bis 10 Kohlenstoffatomen steht,
herstellbar durch ein Verfahren bei dem
   a) ein Keton der allgemeinen Formel (II) 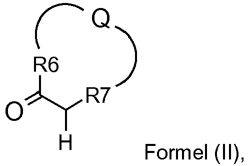 wobei die Reste R6, R7 und Q identisch sind zur allgemeinen Formel (I), in Gegenwart eines Phosphonats der allgemeinen Formel (III) 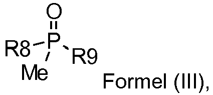 wobei R8 und R9 jeweils unabhängig voneinander für eine lineare oder verzweigte, substituierte oder unsubstituierte Alkoxygruppe mit 1 bis 15 Kohlenstoffatomen stehen, mit einem Benzonitril der allgemeinen Formel (IV) 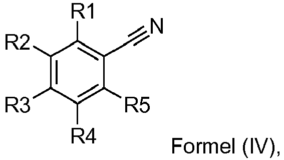 wobei die Reste R1, R2, R3, R4 und R5 identisch sind zur allgemeinen Formel (I), umgesetzt wird und anschließend
   b) das unter Schritt a) erhaltene alpha,beta-ungesättigte Keton der allgemeinen Formel (V) 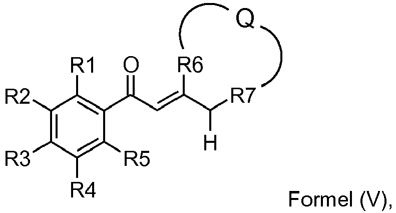 wobei die Reste R1 bis R7 und Q identisch sind zur allgemeinen Formel (I),
      selektiv zu einem Keton der allgemeinen Formel (I) hydriert wird, wobei das Keton der allgemeinen Formel (I) ein Keton der allgemeinen Formel (VI) ist.

Ein Mittel, enthaltend ein Keton der allgemeinen Formel (I) liefert auch nach langer Lagerzeit einen angenehmen Dufteindruck, da die in den Duftspeicherstoffen gespeicherten Duftstoffe erst durch Bestrahlung mit vorzugsweise natürlichem Sonnenlicht oder handelsüblichen Leuchtmitteln wieder freigesetzt werden. So wird ein vorzeitiges Abdampfen des Duftstoffs verhindert.

In einer bevorzugten Ausführungsform der Erfindung handelt es sich bei dem Mittel um ein Wasch- oder Reinigungsmittel, kosmetisches Mittel, Klebstoff oder eine Druckfarbe.

Insbesondere bei Wasch- oder Reinigungsmitteln ist ein frischer Dufteindruck vom Konsumenten ausdrücklich gewünscht. Ein Wasch- oder Reinigungsmittel enthaltend ein Keton der allgemeinen Formel (VI) ist deshalb besonders vorteilhaft, da das Keton ein Limonenspeicher ist und der Geruch von Limonen vom Konsumenten mit Frische verbunden wird.

In einer weiteren bevorzugten Ausführungsform der Erfindung enthält das Mittel das Keton der allgemeinen Formel (I) in Mengen zwischen 0,0001 und 10 Gew.-%, vorteilhafterweise zwischen 0,0005 und 5 Gew.-%, weiter vorteilhaft zwischen 0,001 und 3 Gew.-%, insbesondere zwischen 0,005 und 2 Gew.-%, Gew.-% jeweils bezogen auf das gesamte Mittel.

Da ein Keton der allgemeinen Formel (I) einen erheblich niedrigeren Dampfdruck besitzt als sein korrespondierender Duftstoff, können geringere Mengen des Duftstoffspeichers eingesetzt werden als des Duftstoffs selbst, um einen lange anhaltenden Dufteffekt zu erzielen, was aus ökologischer und ökonomischer Sicht vorteilhaft ist.

Offenbart ist die Verwendung eines Ketons der allgemeinen Formel (I) 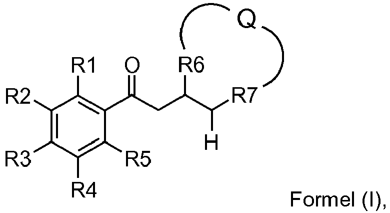
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können und
R6 und R7 unabhängig voneinander für ein sekundäres, tertiäres oder quartäres C-Atom stehen und
Q für eine R6 und R7 verbrückende substituierte oder unsubstituierte Gruppe mit 1 bis 10 Kohlenstoffatomen steht,
herstellbar durch das erfindungsgemäße Verfahren,
in einem Wasch- oder Reinigungsmittel, kosmetischen Mittel, Klebstoff oder einer Druckfarbe zur Verlängerung des Dufteindrucks des Wasch- oder Reinigungsmittels, kosmetischen Mittels, Klebstoffs oder der Druckfarbe.

Ebenfalls offenbart ist die Verwendung eines Ketons der allgemeinen Formel (I) 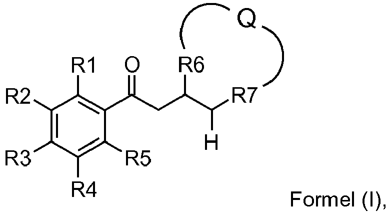
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können und
R6 und R7 unabhängig voneinander für ein sekundäres, tertiäres oder quartäres C-Atom stehen und
Q für eine R6 und R7 verbrückende substituierte oder unsubstituierte Gruppe mit 1 bis 10 Kohlenstoffatomen steht,
herstellbar durch das erfindungsgemäße Verfahren,
zur Verlängerung des Dufteindrucks auf einer mit einem Wasch- oder Reinigungsmittel, kosmetischen Mittel, Klebstoff oder Druckfarbe behandelten Oberfläche.

Im Folgenden soll die Erfindung, unter anderem anhand von Beispielen, eingehender erläutert werden.

Bei dem erfindungsgemäßen Verfahren zur Herstellung eines Ketons der allgemeinen Formel (I) handelt es sich um ein zweistufiges Verfahren, bei dem zunächst ein Keton der allgemeinen Formel (II) in Gegenwart eines Phosphonats der allgemeinen Formel (III) mit einem Benzonitril der allgemeinen Formel (IV) umgesetzt wird und anschließend das erhaltene alpha,beta-ungesättigte Keton der allgemeinen Formel (V) selektiv zu einem Keton der allgemeinen Formel (I) hydriert wird.

Vorzugsweise wird die Umsetzung des Ketons der allgemeinen Formel (II) mit dem Phosphonat der allgemeinen Formel (III) und dem Nitril der allgemeinen Formel (IV) in einem Lösungsmittel durchgeführt. Geeignete Lösungsmittel sind aprotisch polare Lösungsmittel wie beispielsweise Tetrahydrofuran (THF), Diethylether, Aceton, Dichlormethan, Dimethylformamid (DMF), Ethylenglycoldimethylether (DME) oder Gemische davon. Insbesondere THF, Diethylether und DME sind besonders bevorzugte Lösungsmittel im Sinne dieser Erfindung.

Die Reaktion wird vorzugsweise in Gegenwart einer Base durchgeführt. Als Base kann eine dem Fachmann bekannte Base eingesetzt werden, die das Phosphonat der allgemeinen Formel (III) deprotonieren kann. Bevorzugt werden starke Basen wie beispielsweise Alkalimetallhydride, Alkalimetall-bis(trimethylsilyl)amide, Alkalimetallalkoholate oder n-Butyllithium eingesetzt, wobei die Base nicht auf die hier genannten Beispiele beschränkt ist. Besonders bevorzugt im Sinne dieser Erfindung ist n-Butyllithium.

Die Reaktionstemperatur liegt vorzugsweise zwischen -100 °C und 100 °C und wird entsprechend der Reaktivität der einzelnen Reagenzien angepasst. Dabei kann die Temperatur der Reaktionslösung beispielsweise zunächst -78 °C betragen und im Verlauf der Reaktion schrittweise auf beispielsweise Raumtemperatur, also 20 bis 25 °C, erhöht werden.

Ein Nitril der allgemeinen Formel (IV) ist bevorzugt, wenn die Reste R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen, oder R1 und R2 oder R2 und R3 für einen verbrückenden substituierten oder unsubstituierten Cycloalkyl-, Cycloalkenyl-, Aryl- oder Heterocyclus-Rest stehen.

In einer weiter bevorzugten Ausführungsform der Erfindung stehen vier der fünf Arylsubstituenten R1, R2, R3, R4 und R5 für Wasserstoff. Vorzugsweise stehen R1, R2, R4 und R5 für Wasserstoff, während der Substituent in para-Position R3 für ein Halogenatom, insbesondere -F, -Cl, -Br oder -I, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen steht. In einer überaus bevorzugten Ausführungsform der Erfindung steht R3 für -Cl, -Br, -NO₂ oder eine Alkyl- oder Alkoxygruppe umfassend 1 bis 4 C-Atome. Vorzugsweise handelt es sich bei der linearen oder verzweigten, substituierten oder unsubstituierten Alkylgruppe um eine Methyl- oder Ethylgruppe und/oder bei der linearen oder verzweigten, substituierten oder unsubstituierten Alkoxygruppe um eine Methoxy-, Ethoxy-, Isopropoxy- oder tert-Butoxy-Gruppe, wobei eine Methoxygruppe ganz besonders bevorzugt ist.

Eine Substitution in para-Position R3 ist besonders bevorzugt, da die elektronische Struktur des aromatischen Rings hier am effektivsten modifiziert werden kann, wodurch das Absorptionsmaximum von einem Keton der allgemeinen Formel (I) leicht an eine bestimmte Wellenlänge angepasst werden kann.

Ebenfalls bevorzugt ist ein Keton der allgemeinen Formel (I), in dem R1, R2, R3, R4 und R5 für Wasserstoff stehen.

Das Nitril der allgemeinen Formel (IV) wird in Bezug auf das Phosphonat der allgemeinen Formel (III) in einem Verhältnis von vorzugsweise 1:5 bis 5:1, weiter bevorzugt 1:2 bis 2:1 und besonders bevorzugt 1:1,5 bis 1,5:1 eingesetzt.

Ein Keton der allgemeinen Formel (II) ist bevorzugt, wenn die Reste R6 und R7 unabhängig voneinander für ein sekundäres, tertiäres oder quartäres C-Atom stehen und Q für eine R6 und R7 verbrückende substituierte oder unsubstituierte Gruppe mit 1 bis 10 Kohlenstoffatomen steht.

In einer weiter bevorzugten Ausführungsform der Erfindung stehen R6 und R7 in Formel (II) unabhängig voneinander für ein sekundäres oder tertiäres Kohlenstoffatom. In einer ganz besonders bevorzugten Ausführungsform der Erfindung steht einer der beiden Reste R6 und R7 für ein sekundäres Kohlenstoffatom, während der jeweils andere Rest R6 oder R7 für ein tertiäres Kohlenstoffatom steht.

In einer weiteren bevorzugten Ausführungsform der Erfindung steht Q für eine R6 und R7 verbrückende substituierte oder unsubstituierte Gruppe, wobei der R6 und R7 verbrückende Teil von Q so gewählt ist, dass ein vier-, fünf-, sechs-, sieben- oder acht-gliedriger Ring vorliegt. Q ist besonders bevorzugt, wenn der R6 und R7 verbrückende Teil von Q so gewählt ist, dass ein fünf- oder sechs-gliedriger Ring vorliegt.

In einer weiteren bevorzugten Ausführungsform der Erfindung enthält das Keton der allgemeinen Formel (II) mindestens eine semicyclische oder exocyclische Doppelbindung. Die Speicherung eines Duftstoffs mit mindestens einer cyclischen Doppelbindung über diese cyclische Doppelbindung, wobei der Duftstoff mindestens eine zusätzliche semicyclische oder exocyclische Doppelbindung aufweist, stellt eine besondere Herausforderung dar, da häufig keine ausreichende Selektivität zwischen den Doppelbindungen erzielt werden kann oder der Duftstoff sogar selektiv über die semicyclische oder exocyclische Doppelbindung gespeichert wird. Diese unerwünschten Isomere werden bei Bestrahlung mit natürlichem Sonnenlicht oder einem kommerziell erhältlichen Leuchtmittel gar nicht, oder zumindest nicht in ausreichender Geschwindigkeit gespalten, so dass der Dufteindruck einer Mehrkomponenten-Parfümölmischung entscheidend verändert werden kann. Das erfindungsgemäße Verfahren ermöglicht dagegen die selektive Speicherung eines Duftstoffs mit mindestens einer cyclischen Doppelbindung über seine cyclische Doppelbindung, unabhängig von der Anzahl und der chemischen Eigenschaften weiterer semicyclischer oder exocyclischer Doppelbindungen.

Der verbrückende Teil -CH-R7-Q-R6- des Ketons der allgemeinen Formel (II) ist vorzugsweise ein Kohlenwasserstoff, wie bereits oben beschrieben.

In einer ganz besonders bevorzugten Ausführungsform der Erfindung ist das Keton der allgemeinen Formel (II) Dihydrocarvon.

Das Keton der allgemeinen Formel (II) wird in Bezug auf das Phosphonat der allgemeinen Formel (III) in einem Verhältnis von vorzugsweise 1:20 bis 20:1, weiter bevorzugt von 1:10 bis 10:1, noch weiter bevorzugt 1:5 bis 5:1 und am meisten bevorzugt 1:2 bis 2:1 eingesetzt.

Die Aufreinigung des gebildeten alpha,beta-ungesättigten Ketons der allgemeinen Formel (V) erfolgt vorzugsweise durch Destillation, Kristallisation und/oder chromatographische Trennverfahren, die dem Fachmann bekannt sind.

Die Hydrierung des alpha,beta-ungesättigten Ketons der allgemeinen Formel (V) erfolgt vorzugsweise in Gegenwart eines Katalysators und eines Alkalimetallborhydrids und führt selektiv zu einem Keton der allgemeinen Formel (I). Vorzugsweise ist der Katalysator auf Aktivkohle adsorbiertes Palladium, wie es im Stand der Technik hinlänglich bekannt ist. Ein besonders bevorzugtes Alkalimetallborhydrid ist Natriumborhydrid.

Die Hydrierung erfolgt bevorzugt in einem Lösungsmittel. Geeignete Lösungsmittel können beispielsweise ausgewählt sein aus der Gruppe der Alkohole, Wasser, Carbonsäureester, Halogenalkane, aromatischen Lösungsmittel oder Gemischen daraus, wobei Toluol ein besonders bevorzugtes Lösungsmittel im Sinne dieser Erfindung ist.

Die Hydrierung des alpha,beta-ungesättigten Ketons der allgemeinen Formel (V) findet vorzugsweise bei Raumtemperatur, also 20 bis 25 °C, und Normaldruck, also 0,9 bis 1,1 bar, statt. Gegebenenfalls kann es aber bevorzugt sein die Hydrierung bei tieferen oder höheren Temperaturen und/oder insbesondere höherem Druck durchzuführen.

Ein weiterer Gegenstand der Erfindung ist ein Keton der allgemeinen Formel (VI), das durch Umsetzung von
a) Dihydrocarvon mit einem Benzonitril der allgemeinen Formel (IV) in Gegenwart eines Phosphonats der allgemeinen Formel (III), und anschließender
b) selektiver Hydrierung des in Schritt a) erhaltenen alpha,beta-ungesättigten Ketons zu einem Keton der allgemeinen Formel (VI)
erhalten werden kann.

Ketone der allgemeinen Formel (VI) konnten bisher nicht selektiv hergestellt werden, sind aus parfümistischer Sicht jedoch besonders interessant, da der gespeicherte Duftstoff Limonen ist. Limonen ist eine der wichtigsten Basiskomponenten für Mehrkomponenten-Parfümölgemische und vermittelt dem Konsumenten einen frischen Dufteindruck, der mit Reinheit verbunden wird.

Ein weiterer Gegenstand der Erfindung ist ein Mittel enthaltend mindestens eine Verbindung der allgemeinen Formel (I), 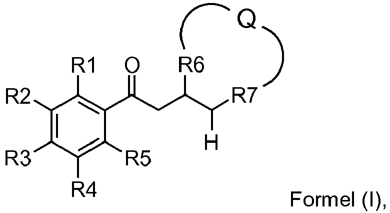
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen,
eine lineare oder verzweigte, substituierte oder unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen,-OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können und R6 und R7 unabhängig voneinander für ein sekundäres, tertiäres oder quartäres C-Atom stehen und
Q für eine R6 und R7 verbrückende substituierte oder unsubstituierte Gruppe mit 1 bis 10 Kohlenstoffatomen steht,
herstellbar durch ein Verfahren bei dem
   a) ein Keton der allgemeinen Formel (II) 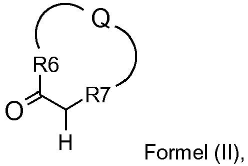 wobei die Reste R6, R7 und Q identisch sind zur allgemeinen Formel (I),
      in Gegenwart eines Phosphonats der allgemeinen Formel (III) 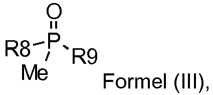 wobei R8 und R9 jeweils unabhängig voneinander für eine lineare oder verzweigte, substituierte oder unsubstituierte Alkoxygruppe mit 1 bis 15 Kohlenstoffatomen stehen, mit einem Benzonitril der allgemeinen Formel (IV) 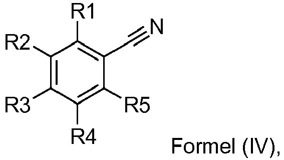 wobei die Reste R1, R2, R3, R4 und R5 identisch sind zur allgemeinen Formel (I), umgesetzt wird und anschließend
   b) das unter Schritt a) erhaltene alpha,beta-ungesättigte Keton der allgemeinen Formel (V) 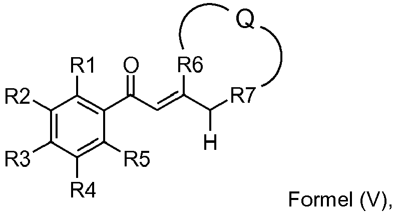 wobei die Reste R1 bis R7 und Q identisch sind zur allgemeinen Formel (I),
      selektiv zu einem Keton der allgemeinen Formel (I) hydriert wird, wobei das Keton der allgemeinen Formel (I) ein Keton der allgemeinen Formel (VI) ist.

In einer bevorzugten Ausführungsform der Erfindung handelt es sich bei dem Mittel um ein Wasch- oder Reinigungsmittel, kosmetisches Mittel, Klebstoff oder eine Druckfarbe, wobei Wasch- oder Reinigungsmittel und kosmetische Mittel besonders bevorzugt sind.

In einer bevorzugten Ausführungsform der Erfindung enthält ein solches Mittel das Keton der allgemeinen Formel (I) in Mengen zwischen 0,0001 und 10 Gew.-%, vorteilhafterweise zwischen 0,0005 und 5 Gew.-%, weiter vorteilhaft zwischen 0,001 und 3 Gew.-%, insbesondere zwischen 0,005 und 2 Gew.-%, Gew.-% jeweils bezogen auf das gesamte Mittel.

In einer bevorzugten Ausführungsform der Erfindung enthält ein Mittel enthaltend ein Keton der allgemeinen Formel (I), wie z.B. Wasch- oder Reinigungsmittel, kosmetisches Mittel, Klebstoff oder Druckfarbe mindestens einen weiteren Duftstoff. Die bevorzugt eingesetzten Duftstoffe bzw. Parfümöle sind keinerlei Beschränkungen unterworfen. So können vorzugsweise als Duftstoffe synthetische oder natürliche Duftstoffverbindungen vom Typ der Ester, Ether, Aldehyde (Duftaldehyde), Ketone (Duftketone), Alkohole, Kohlenwasserstoffe, Säuren, Kohlensäureester, aromatischen Kohlenwasserstoffe, aliphatischen Kohlenwasserstoffe, gesättigten und / oder ungesättigten Kohlenwasserstoffe und Mischungen daraus verwendet werden.

Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann allgemein bekannt. Die Duftketone können alle Ketone umfassen, die einen erwünschten Duft oder ein Frischeempfinden verleihen können. Es können auch Gemische unterschiedlicher Ketone eingesetzt werden. Einsetzbare Ketone sind z.B. alpha-Damascon, delta-Damascon, Iso-Damascon, Carvon, gamma-Methylionon, Iso-E-Super, 2,4,4,7-Tetramethyl-oct-6-en-3-on, Benzylaceton, Beta Damascon, Damascenon, Methyldihydrojasmonat, Methylcedrylon, Hedion und Gemischen davon. Geeignete Duftaldehyde können beliebige Aldehyde sein, die entsprechend der Duftketone einen gewünschten Duft oder ein Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Lilial usw. Die Duftaldehyde und Duftketone können eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen. Geeignete Duftstoffe vom Typ der Ester sind beispielsweise Benzylacetat, Phenoxyethylisobutyrat, p-tert-Butylcyclohexylacetat und so weiter. Duftstoffverbindungen vom Typ der Kohlenwasserstoffe sind beispielsweise Terpene wie Limonen und Pinen. Geeignete Duftstoffe vom Typ Ether sind beispielsweise Benzylethylether und Ambroxan. Geeignete Duftstoffalkohole sind beispielsweise 10-Undecen-1-ol, 2,6-Dimethylheptan-2-ol, 2-Methylbutanol, 2-Methylpentanol, 2-Phenoxyethanol, 2-Phenylpropanol und so weiter. Duftstoffe bzw. Parfümöle können auch natürliche Duftstoffgemische sein, wie sie aus pflanzlichen Quellen zugänglich sind. Bei den Duftstoffen beziehungsweise Parfümölen kann es sich auch um ätherische Öle, wie zum Beispiel Angelikawurzelöl, Anisöl, Arnikablütenöl und so weiter handeln. Die Gesamtmenge des mindestens einen weiteren Duftstoffs in dem Mittel, wie zum Beispiel Wasch- oder Reinigungsmittel, kosmetisches Mittel, Klebstoff oder Druckfarbe beträgt vorzugsweise zwischen 0,001 und 5 Gew.-%, vorteilhafterweise zwischen 0,01 und 4 Gew.-%, weiter vorteilhaft zwischen 0,1 und 3 Gew.-% sowie ganz besonders bevorzugt zwischen 0,5 und 2 Gew.-% bezogen auf das gesamte Mittel. Bevorzugt werden Mischungen verschiedener Duftstoffe aus den verschiedenen oben genannten Duftstoffklassen verwendet, die gemeinsam eine ansprechende Duftnote erzeugen.

In einer bevorzugten Ausführungsform der Erfindung enthalten die Mittel, wie. z.B. Wasch- oder Reinigungsmittel, kosmetische Mittel, Klebstoffe oder Druckfarben, mindestens ein Tensid, vorzugsweise ausgewählt aus der Gruppe bestehend aus anionischen, kationischen, nichtionischen, zwitterionischen, amphoteren Tensiden oder deren Mischungen.

Ein bevorzugtes Mittel kann fest oder flüssig sein, wobei flüssige Mittel, insbesondere Wasch- oder Reinigungsmittel oder Waschhilfsmittel, bevorzugt sind. Insbesondere für den Fall, dass das Mittel ein Wasch- oder Reinigungsmittel ist, ist es bevorzugt, dass es mindestens ein Tensid ausgewählt aus der Gruppe bestehend aus anionischen, nichtionischen, zwitterionischen und amphoteren Tensiden enthält. Insbesondere für den Fall, dass das Mittel ein weichmachendes Waschmittel ("2in1") ist, ist es bevorzugt, dass es eine weichmachende Komponente sowie mindestens ein Tensid ausgewählt aus der Gruppe bestehend aus anionischen, nichtionischen, zwitterionischen und amphoteren Tensiden enthält. Waschhilfsmittel werden zur gezielten Vorbehandlung der Wäsche vor dem Waschen bei Flecken oder starker Verschmutzung eingesetzt. Zu den Waschhilfsmitteln gehören beispielsweise Vorbehandlungsmittel, Einweichmittel, Entfärber und Fleckensalz. Insbesondere für den Fall, dass das Mittel ein Weichspüler ist, ist es bevorzugt, dass es eine weichmachende Komponente enthält. Weichspüler sind als Mittel bevorzugt, da sie erst im letzten Schritt eines konventionellen Textilwaschvorgangs, dem Spülgang, in Kontakt mit den Textilien kommen und somit eine möglichst große Menge der Duftstoffe auf das Textil aufziehen können, ohne dass die Gefahr besteht, dass die Duftstoffe bei anschließenden Schritten wieder entfernt werden. Es ist ganz besonders bevorzugt, dass die weichmachende Komponente eine alkylierte, quaternäre Ammoniumverbindung ist, wobei mindestens eine Alkylkette durch eine Ester- oder Amidogruppe unterbrochen ist. Die weichmachende Komponente umfasst beispielsweise quaternäre Ammoniumverbindungen wie Monoalk(en)yltrimethylammonium-Verbindungen, Dialk(en)yldimethyl-ammonium-Verbindungen, Mono-, Di- oder Triester von Fettsäuren mit Alkanolaminen. Weitere verwendbare weichmachende Komponenten stellen quaternisierte Proteinhydrolysate oder protonierte Amine dar. Weiterhin sind auch kationische Polymere geeignete weichmachende Komponente. Ebenfalls einsetzbar sind polyquaternierte Polymere (z.B. Luviquat® Care von BASF) und auch kationische Biopolymere auf Chitinbasis und deren Derivate, beispielsweise das unter der Handelsbezeichnung Chitosan® (Hersteller: Cognis) erhältliche Polymer. Weitere geeignete weichmachende Komponenten umfassen protonierte oder quaternierte Polyamine. Besonders bevorzugte weichmachende Komponenten sind alkylierte quaternäre Ammoniumverbindungen, von denen mindestens eine Alkylkette durch eine Estergruppe und/oder Amidogruppe unterbrochen ist. Ganz besonders bevorzugt sind N-Methyl-N-(2-hydroxy-ethyl)-N,N-(ditalgacyloxyethyl)ammonium-methosulfat oder Bis-(palmitoyloxyethyl)-hydroxyethylmethylammoniummethosulfat.

Das Mittel, insbesondere in Form von Weichspülern, kann auch nichtionische weichmachende Komponenten enthalten, wie vor allem Polyoxyalkylenglycerolalkanoate, Polybutylene, langkettige Fettsäuren, ethoxylierte Fettsäureethanolamide, Alkylpolyglucoside, insbesondere Sorbitanmono,di- und -triester, und Fettsäureester von Polycarbonsäuren. In dem Weichspüler als Mittel ist vorteilhafterweise die weichmachende Komponente in Mengen von 0,1 bis 80 Gew.-%, üblicherweise 1 bis 40 Gew.-%, vorzugsweise 2 bis 20 Gew.-% und insbesondere 3 bis 15 Gew.-% und der mindestens eine Duftstoff oder die Mischung verschiedener Duftstoffe in Mengen von vorteilhafterweise 0,1 bis 20 Gew.-%, vorzugsweise 1 bis 13 Gew.-% und insbesondere 2 bis 8 Gew.-%, jeweils bezogen auf die Gesamtmenge des Mittels, enthalten.

Als weitere Komponente kann das Mittel gegebenenfalls ein oder mehrere nichtionische Tenside enthalten, wobei solche eingesetzt werden können, die üblicherweise auch in Waschmitteln verwendet werden.

Es ist weiterhin bevorzugt, dass das Mittel, insbesondere in Form eines Wasch- oder Reinigungsmittels, zusätzlich weitere vorteilhafte Inhaltsstoffe enthält, die dem Fachmann bekannt sind. So kann das Mittel, wie insbesondere Wasch- oder Reinigungsmittel, zusätzlich zu den Tensiden und/oder weichmachenden Verbindungen weitere Inhaltsstoffe enthalten, die die anwendungstechnischen und/oder ästhetischen Eigenschaften des Mittels weiter verbessern. Im Rahmen der vorliegenden Erfindung enthalten bevorzugte Mittel zusätzlich einen oder mehrere Stoffe aus der Gruppe der Gerüststoffe, Bleichmittel, Bleichaktivatoren, Enzyme, Elektrolyte, nichtwässrigen Lösungsmittel, pH-Stellmittel, Profragrances, Fluoreszenzmittel, Farbstoffe, Hydrotope, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber. Als Gerüststoffe, die in den Mitteln enthalten sein können, sind insbesondere Silikate, Aluminiumsilikate wie insbesondere Zeolithe, Carbonate, Salze organischer Di- und Polycarbonsäuren sowie Mischungen dieser Stoffe zu nennen.

Als Reinigungsmittel kann das Mittel z.B. zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Als harte Oberflächen kommen neben Geschirr auch alle übrigen harten Oberflächen, insbesondere aus Glas, Keramik, Kunststoff, Holz oder Metall, in Haushalt und Gewerbe in Frage. Dabei kann es sich, wie auch bei allen anderen Mitteln, um feste oder flüssige Formulierungen handeln, wobei feste Formulierungen als Pulver, Granulat, Extrudat, in Tab-Form, als Tablette oder als gepresster und/oder geschmolzener Formkörper vorliegen können. Bei flüssigen Formulierungen kann es sich um Lösungen, Emulsionen, Dispersionen Suspensionen, Mikroemulsionen, Gels oder Pasten handeln.

Die Herstellung fester Mittel, das heißt Wasch- oder Reinigungsmittel, bereitet keine Schwierigkeiten und kann im Prinzip bekannter Weise, zum Beispiel durch Sprühtrocknen oder Granulation, erfolgen, wobei optionale Persauerstoffverbindungen und optionaler Bleichkatalysator gegebenenfalls später zugesetzt werden. Zur Herstellung von Mitteln mit erhöhtem Schüttgewicht, insbesondere im Bereich von 650 g/L bis 950 g/L, ist ein einen Extrusionsschritt aufweisendes Verfahren bevorzugt. Die Herstellung flüssiger Mittel bereitet ebenfalls keine Schwierigkeiten und kann ebenfalls in bekannter Weise erfolgen. Die Einarbeitung der Duftspeicherstoffe kann insbesondere zusammen mit anderen Duftstoffen erfolgen.

Ferner ist die Verwendung eines Ketons der allgemeinen Formel (I) offenbart, herstellbar durch das erfindungsgemäße Verfahren, in einem Wasch- oder Reinigungsmittel, kosmetischen Mittel, Klebstoff oder einer Druckfarbe zur Verlängerung des Dufteindrucks des Wasch- oder Reinigungsmittels, kosmetischen Mittels, Klebstoffs oder der Druckfarbe. Durch die Verwendung des Ketons der allgemeinen Formel (I) in einem oben beschriebenen Mittel wird ein möglicherweise unangenehmer Geruch anderer Inhaltsstoffe eines oben beschriebenen Mittels über einen langen Zeitraum effektiv maskiert.

Ebenfalls offenbart ist die Verwendung eines Ketons der allgemeinen Formel (I), herstellbar durch das erfindungsgemäße Verfahren, zur Verlängerung des Dufteindrucks auf einer mit einem Wasch- oder Reinigungsmittel, kosmetischen Mittel, Klebstoff oder Druckfarbe behandelten Oberfläche. Ein Keton der allgemeinen Formel (I) erzeugt auf einer mit dem Keton behandelten harten oder weichen Oberfläche einen verlängerten, vom Konsumenten als angenehm und frisch wahrgenommenen Dufteindruck, der insbesondere bei Wasch- oder Reinigungsmitteln mit Sauberkeit und Reinheit verbunden wird.

### Beispiele

### Herstellung von Diethylmethylphosphonat

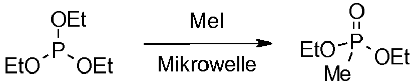

Es wurden 17,4 mL (100 mmol, 1 eq) Triethylphosphit und 11,3 mL (180 mmol, 1,8 eq) lodmethan in ein Mikrowellenvial gegeben und dieses versiegelt. Innerhalb von 5 min wurde bei einer Leistung von 50 W auf 120 °C erhitzt und diese Temperatur für weitere 5 min gehalten. Im Anschluss wurde überschüssiges lodmethan und entstandenes lodethan bei 40 °C im Hochvakuum entfernt, wodurch 14.9 g (98%) Diethylmethylphosphonat erhalten wurden.
M (C₅H₁₃O₃P) = 152.13 g/mol
**¹H-NMR** (CDCl₃, 500 MHz): δ [ppm] = 3.65-3.53 (m, 4x H aus CH₂), 0.97 (d, J = 17.5 Hz, 3x H aus CH₃ an P), 0.83 (t, J = 7.2 Hz, 3x H aus Et).
**¹³C-NMR** (CDCl₃, 125 MHz): δ [ppm] = 60.3 (2x CH₂), 15.4 (2x CH₃), 10.1 (1x CH₃).
**³¹P-NMR** (CDCl₃, 200 MHz): δ [ppm] = 31.2 (1x P).
**MS** (EtOAc, EI): m/z [%] = 152 [M⁺] (4%), 137 (9%), 125 (86%), 123 (13%), 109 (9%), 108 (24%), 107 (24%), 97 (100%), 93 (6%), 91 (6%), 81 (18%), 80 (35%), 79 (89%), 65 (14%).

### Herstellung von 1-Benzoylmethylenmenthen

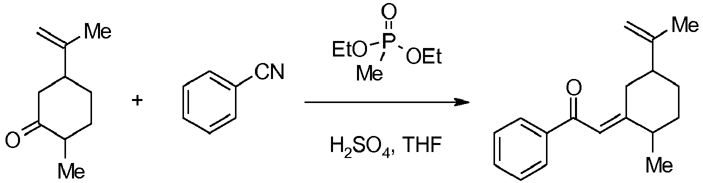

3.9 mL (26.3 mmol, 1 eq) Diethylmethylphosphonat wurden in 130 mL THF gelöst, die Lösung auf -78 °C gekühlt, 20.0 mL (31.6 mmol, 1.2 eq) *n*-BuLi (c = 1.6 mol/L) zugetropft und die Mischung 90 min gerührt. Dann wurden 3.0 mL (28.9 mmol, 1.1 eq) Benzonitril zugetropft und die Mischung innerhalb von 90 min auf -15 °C auftauen gelassen. Dann wurden 2.2 mL (13.2 mmol, 0.5 eq) Dihydrocarvon zugetropft, die Mischung für weitere 30 min bei -15 °C gerührt und danach über Nacht bei Raumtemperatur gerührt. Schließlich wurden 10 mL Schwefelsäure (c = 2.5 mol/L) zugegeben, weitere 60 min gerührt, 30 mL Wasser zugegeben, die Phasen separiert, die wässrige Phase zweimal mit 50 mL Diethylether extrahiert, die vereinigten organischen Phasen mit 50 mL gesättigter Natriumchloridlösung gewaschen und über Magnesiumsulfat getrocknet. Das Lösungsmittel wurde im Vakuum entfernt, wodurch das Rohprodukt als Diastereomerengemisch erhalten wurde.
M (C₁₈H₂₂O) = 254.37 g/mol
**MS** (EtOAc, EI): m/z [%] = 254 [M⁺] (57%), 211 (47%), 197 (9%), 178 (10%), 165 (11%), 149 (12%), 134 (11%), 128 (11%), 119 (25%), 115 (17%), 107 (20%), 106 (16%), 105 (100%), 94 (13%), 93 (18%), 91 (32%), 79 (16%), 78 (17%), 77 (45%), 51 (10%).

### Herstellung von 3-Phenacylmenthen

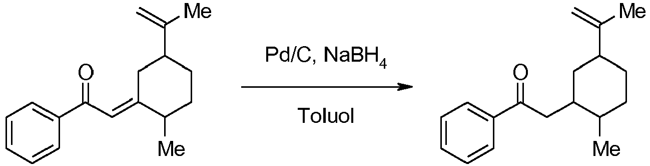

1.0 g (3.9 mmol, 1 eq) 1-Benzoylmethylenmenthen wurden in 15 mL Toluol gelöst, 0.5 mL (7.9 mmol, 2 eq) Essigsäure und 106 mg (98.3 µmol, 0.025 eq) Pd/C (10%) zugegeben und schließlich 0.6 g (15.7 mmol, 4 eq) Natriumborhydrid zugesetzt. Es wurde eine Stunde gerührt, dann wurde überschüssiges Natriumborhydrid mit Salzsäure (c = 0.1 mol/L) desaktiviert, mit gesättigter Natriumhydrogencarbonatlösung neutralisiert, der Katalysator über einen Feinfilter abfiltriert, die Phasen separiert, die wässrige Phase zweimal mit 50 mL Diethylether extrahiert, die vereinigten organischen Phasen mit 50 mL Wasser gewaschen und über Magnesiumsulfat getrocknet. Das Lösungsmittel wurde im Vakuum entfernt, wodurch 756 mg Rohprodukt von 3-Phenacylmenthen erhalten wurden.
M (C₁₈H₂₄O) = 256.38 g/mol
**MS** (EtOAc, EI): m/z [%] = 256 [M⁺] (8%), 238 (12%), 213 (10%), 207 (13%), 181 (10%), 170 (13%), 145 (12%), 136 (34%), 128 (11%), 121 (55%), 120 (85%), 119 (24%), 107 (22%), 105 (100%), 95 (15%), 94 (37%), 93 (42%), 92 (16%), 91 (35%), 81 (18%), 79 (30%), 78 (25%), 77 (70%), 67 (22%), 51 (16%).

### Belichtung von 3-Phenacylmenthen

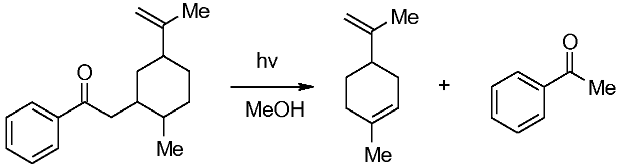

110 mg 3-Phenacylmenthen wurden in 10 mL Methanol gelöst (c = 4 x 10⁻² mol/L) und bei lambda-ₘₐₓ = 300 nm 18 h belichtet. Die Reaktion wurde mittels Gaschromatographie verfolgt. Nach 18 h ist das 3-Phenacylmenthen weitgehend gespalten, es wurden neue Signale mittels NMR detektiert, die Limonen und Acetophenon zuzuordnen sind.

## Patentansprüche

1. Verfahren zur Herstellung eines Ketons der allgemeinen Formel (I) 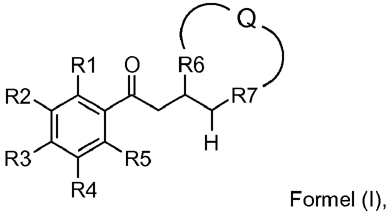
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder
unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können und
R6 und R7 unabhängig voneinander für ein sekundäres, tertiäres oder quartäres C-Atom stehen und
Q für eine R6 und R7 verbrückende substituierte oder unsubstituierte Gruppe mit 1 bis 10 Kohlenstoffatomen steht,
**dadurch gekennzeichnet, dass**
a) ein Keton der allgemeinen Formel (II) 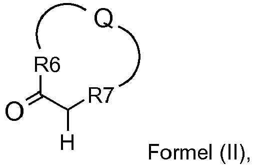 wobei die Reste R6, R7 und Q identisch sind zur allgemeinen Formel (I), in Gegenwart eines Phosphonats der allgemeinen Formel (III) 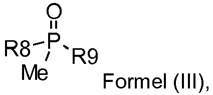 wobei R8 und R9 jeweils unabhängig voneinander für eine lineare oder verzweigte, substituierte oder unsubstituierte Alkoxygruppe mit 1 bis 15 Kohlenstoffatomen stehen, mit einem Benzonitril der allgemeinen Formel (IV) 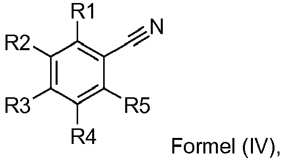 wobei die Reste R1, R2, R3, R4 und R5 identisch sind zur allgemeinen Formel (I), umgesetzt wird und anschließend
b) das unter Schritt a) erhaltene alpha,beta-ungesättigte Keton der allgemeinen Formel (V) 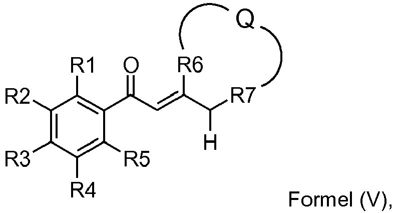 wobei die Reste R1 bis R7 und Q identisch sind zur allgemeinen Formel (I),
selektiv zu einem Keton der allgemeinen Formel (I) hydriert wird.

2. Verfahren nach Anspruch 1, **dadurch gekennzeichnet, dass** das Keton der allgemeinen Formel (II) mindestens eine semicyclische oder exocyclische Doppelbindung aufweist.

3. Verfahren nach einem der Ansprüche 1 oder 2, **dadurch gekennzeichnet, dass** der verbrückende Teil -CH-R7-Q-R6- des Ketons der allgemeinen Formel (II) ein Kohlenwasserstoff ist.

4. Verfahren nach einem der Ansprüche 1 bis 3, **dadurch gekennzeichnet, dass** das Keton der allgemeinen Formel (II) Dihydrocarvon ist.

5. Verfahren nach einem der Ansprüche 1 bis 4, **dadurch gekennzeichnet, dass** die Reste R8 und R9 des Phosphonats der allgemeinen Formel (III) jeweils unabhängig voneinander Methoxy-, Ethoxy-, Propoxy-, Butoxy- oder Isopropoxy-Reste sind.

6. Verfahren nach einem der Ansprüche 1 bis 5, **dadurch gekennzeichnet, dass** die Reste R1 bis R5 jeweils unabhängig voneinander für Wasserstoff, Methyl- oder Methoxygruppen stehen, wobei Methoxygruppen besonders bevorzugt sind.

7. Verfahren nach einem der Ansprüche 1 bis 6, **dadurch gekennzeichnet, dass** das Hydriermittel ausgewählt ist aus der Gruppe der Alkalimetallborhydride und besonders bevorzugt Natriumborhydrid ist.

8. Keton der allgemeinen Formel (VI), 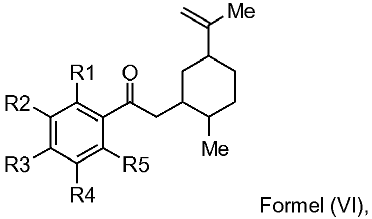
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder
unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können,
herstellbar durch
a) die Umsetzung von Dihydrocarvon mit einem Benzonitril der allgemeinen Formel (IV) in Gegenwart eines Phosphonats der allgemeinen Formel (III), und anschließender
b) selektiver Hydrierung des in Schritt a) erhaltenen alpha,beta-ungesättigten Ketons zu einem Keton der allgemeinen Formel (VI).

9. Mittel enthaltend ein Keton der allgemeinen Formel (I) 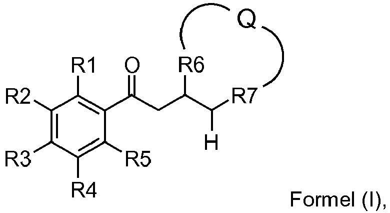
wobei R1, R2, R3, R4 und R5 jeweils unabhängig voneinander, für Wasserstoff, -NO₂, eine lineare oder verzweigte, substituierte oder unsubstituierte Alkoxygruppe mit 1 bis 15 Kohlenstoffatomen, eine lineare oder verzweigte, substituierte oder
unsubstituierte Alkylgruppe mit 1 bis 15 Kohlenstoffatomen, eine Cycloalkyl-, Acyl-, Aryl- oder Heteroarylgruppe mit 1 bis 15 Kohlenstoffatomen, -OH, -NH₂, Halogen, -NH-Alkyl, -N(Alkyl)₂ oder -N⁺(Alkyl)₃ stehen können, oder R1 und R2 oder R2 und R3 für einen verbrückenden substituierten oder unsubstituierten Cycloalkyl-, Cycloalkenyl-, Aryl- oder Heterocyclus-Rest stehen können und
R6 und R7 unabhängig voneinander für ein sekundäres, tertiäres oder quartäres C-Atom stehen und
Q für eine R6 und R7 verbrückende substituierte oder unsubstituierte Gruppe mit 1 bis 10 Kohlenstoffatomen steht,
herstellbar durch ein Verfahren, **dadurch gekennzeichnet, dass**
a) ein Keton der allgemeinen Formel (II) 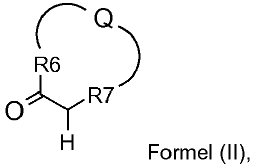 wobei die Reste R6, R7 und Q identisch sind zur allgemeinen Formel (I), in Gegenwart eines Phosphonats der allgemeinen Formel (III) 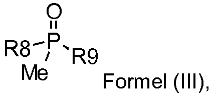 wobei R8 und R9 jeweils unabhängig voneinander für eine lineare oder verzweigte, substituierte oder unsubstituierte Alkoxygruppe mit 1 bis 15 Kohlenstoffatomen stehen, mit einem Benzonitril der allgemeinen Formel (IV) 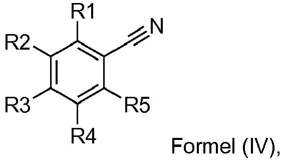 wobei die Reste R1, R2, R3, R4 und R5 identisch sind zur allgemeinen Formel (I), umgesetzt wird und anschließend
b) das unter Schritt a) erhaltene alpha,beta-ungesättigte Keton der allgemeinen Formel (V) 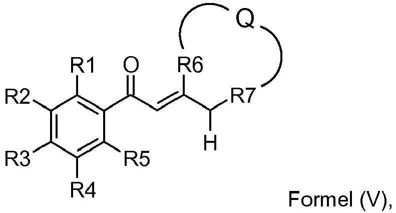 wobei die Reste R1 bis R7 und Q identisch sind zur allgemeinen Formel (I),
selektiv zu einem Keton der allgemeinen Formel (I) hydriert wird,
wobei das Keton der allgemeinen Formel (I) ein Keton der allgemeinen Formel (VI) ist.

10. Mittel nach Anspruch 9, **dadurch gekennzeichnet, dass** es sich bei dem Mittel um ein Wasch- oder Reinigungsmittel, kosmetisches Mittel, Klebstoff oder eine Druckfarbe handelt.

11. Mittel nach einem der Ansprüche 9 bis 10, **dadurch gekennzeichnet, dass** es das Keton der allgemeinen Formel (I) in Mengen zwischen 0,0001 und 10 Gew.-%, vorteilhafterweise zwischen 0,0005 und 5 Gew.-%, weiter vorteilhaft zwischen 0,001 und 3 Gew.-%, insbesondere zwischen 0,005 und 2 Gew.-% enthält, Gew.-% jeweils bezogen auf das gesamte Mittel.

## Claims

1. A method for producing a ketone of the general formula (I) 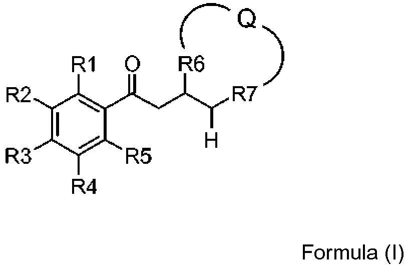
wherein R1, R2, R3, R4 and R5, independently of one another, can each represent hydrogen, -NO₂, a linear or branched, substituted or unsubstituted alkoxy group having 1 to 15 carbon atoms, a linear or branched, substituted or
unsubstituted alkyl group having 1 to 15 carbon atoms, a cycloalkyl,
acyl, aryl or heteroaryl group having 1 to 15 carbon atoms, -OH, -NH₂, halogen, -NH-alkyl, -N(alkyl)₂ or -N⁺(alkyl)₃, or R1 and R2 or R2 and R3 can represent a bridging, substituted or unsubstituted cycloalkyl, cycloalkenyl,
aryl or heterocyclic functional group and
R6 and R7, independently of one another, represent a secondary, tertiary or quaternary carbon atom, and
Q represents an R6 and R7 bridging, substituted or unsubstituted group having from 1 to 10 carbon atoms,
**characterized in that**
a) a ketone of general formula (II) 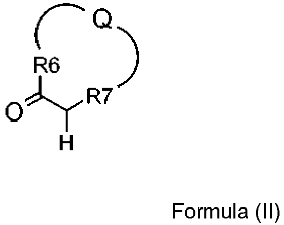 wherein the functional groups R6, R7 and Q are identical to general formula (I), is reacted in the presence of a phosphonate of general formula (III) 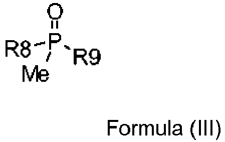 wherein R8 and R9, independently of one another, each represent a linear or branched, substituted or unsubstituted alkoxy group having 1 to 15 carbon atoms, having a benzonitrile of general formula (IV) 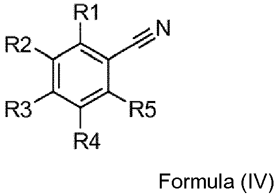 wherein the functional groups R1, R2, R3, R4 and R5 are identical to general formula (I), and subsequently
b) the alpha, beta-unsaturated ketone of general formula (V) obtained in step a) 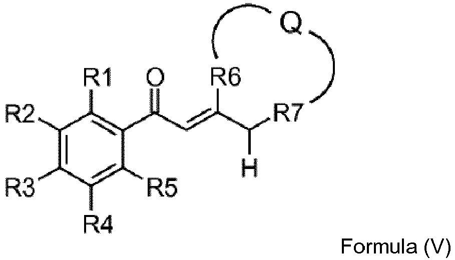 wherein the functional groups R1 to R7 and Q are identical to general formula (I), is selectively hydrogenated to form a ketone of general formula (I).

2. The method according to claim 1, **characterized in that** the ketone of general formula (II) has at least one semicyclic or exocyclic double bond.

3. The method according to one of claims 1 or 2, **characterized in that** the bridging part -CH-R7-Q-R6- of the ketone of general formula (II) is a hydrocarbon.

4. The method according to one of claims 1 to 3, **characterized in that** the ketone of general formula (II) is dihydrocarvone.

5. The method according to one of claims 1 to 4, **characterized in that** the functional groups R8 and R9 of the phosphonate of general formula (III), independently of one another, are each methoxy, ethoxy, propoxy, butoxy or isopropoxy functional groups.

6. The method according to one of claims 1 to 5, **characterized in that** the functional groups R1 to R5, independently of one another, each represent hydrogen, methyl groups or methoxy groups, methoxy groups being particularly preferred.

7. The method according to one of claims 1 to 6, **characterized in that** the hydrogenating agent is selected from the group of alkali metal borohydrides and particularly preferably sodium borohydride.

8. A ketone of general formula (VI), 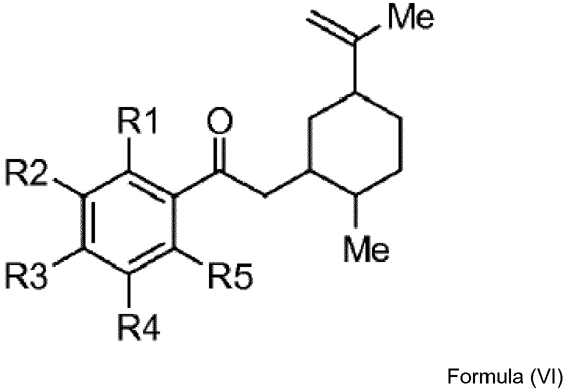
wherein R1, R2, R3, R4 and R5, independently of one another, can each represent hydrogen, -NO₂, a linear or branched, substituted or unsubstituted alkoxy group having 1 to 15 carbon atoms, a linear or branched, substituted or
unsubstituted alkyl group having 1 to 15 carbon atoms, a cycloalkyl,
acyl, aryl or heteroaryl group having 1 to 15 carbon atoms, -OH, -NH₂, halogen, -NH-alkyl, -N(alkyl)₂ or -N⁺(alkyl)₃, or R1 and R2 or R2 and R3 can represent a bridging, substituted or unsubstituted cycloalkyl, cycloalkenyl,
aryl or heterocyclic functional group,
producible by
a) reacting dihydrocarvone with a benzonitrile of general formula (IV) in the presence of a phosphonate of general formula (III) and subsequently
b) selectively hydrogenating the alpha, beta-unsaturated ketone obtained in step a) to form a ketone of general formula (VI).

9. An agent containing a ketone of general formula (I) 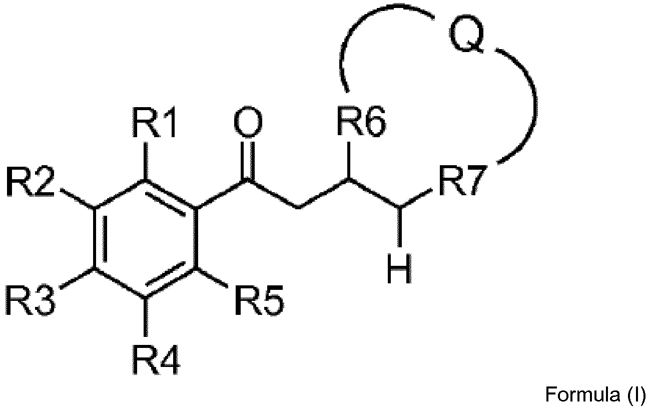
wherein R1, R2, R3, R4 and R5, independently of one another, can each represent hydrogen, -NO₂, a linear or branched, substituted or unsubstituted alkoxy group having 1 to 15 carbon atoms, a linear or branched, substituted or
unsubstituted alkyl group having 1 to 15 carbon atoms, a cycloalkyl,
acyl, aryl or heteroaryl group having 1 to 15 carbon atoms, -OH, -NH₂, halogen, -NH-alkyl, -N(alkyl)₂ or -N⁺(alkyl)₃, or R1 and R2 or R2 and R3 can represent a bridging, substituted or unsubstituted cycloalkyl, cycloalkenyl,
aryl or heterocyclic functional group and
R6 and R7, independently of one another, represent a secondary, tertiary or quaternary carbon atom, and
Q represents an R6 and R7 bridging, substituted or unsubstituted group having from 1 to 10 carbon atoms,
producible by a method, **characterized in that**
a) a ketone of general formula (II) 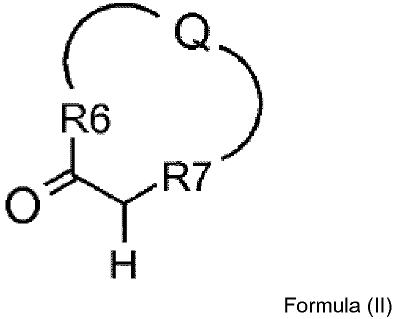 wherein the functional groups R6, R7 and Q are identical to general formula (I), is reacted in the presence of a phosphonate of general formula (III) 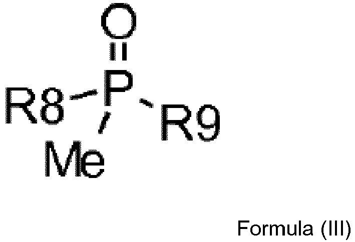 wherein R8 and R9, independently of one another, represent a linear or branched, substituted or unsubstituted alkoxy group having 1 to 15 carbon atoms, having a benzonitrile of general formula (IV) 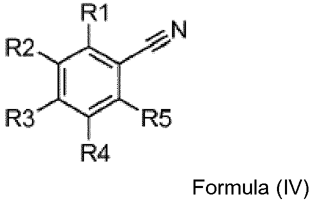 wherein the functional groups R1, R2, R3, R4 and R5 are identical to general formula (I), and subsequently
b) the alpha, beta-unsaturated ketone of general formula (V) obtained in step a) 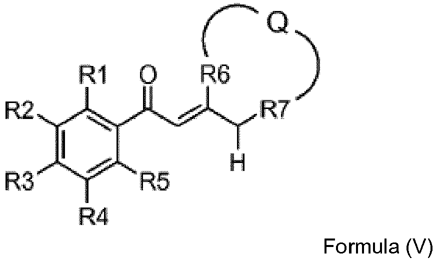 wherein the functional groups R1 to R7 and Q are identical to general formula (I),
is selectively hydrogenated to form a ketone of general formula (I),
wherein the ketone of general formula (I) is a ketone of general formula (VI).

10. The agent according to claim 9, **characterized in that** the agent is a washing or cleaning agent, cosmetic agent, adhesive or printing ink.

11. The agent according to one of claims 9 to 10, **characterized in that** it contains the ketone of general formula (I) in quantities of between 0.0001 and 10 wt.%, preferably between 0.0005 and 5 wt.%, more preferably between 0.001 and 3 wt.%, and in particular between 0.005 and 2 wt.%, the wt.% in each case being based on the total agent.

## Revendications

1. Procédé de préparation d'une cétone de la formule générale (I) 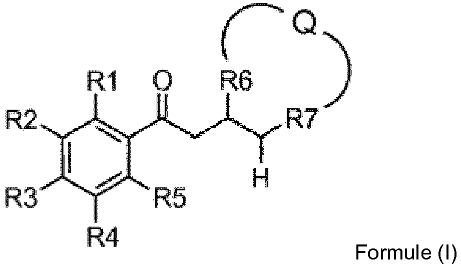
dans laquelle R1, R2, R3, R4 et R5 peuvent représenter chacun indépendamment les uns des autres l'hydrogène, -NO₂, un groupe alcoxy linéaire ou ramifié, substitué ou non substitué, comportant de 1 à 15 atomes de carbone, un groupe alkyle linéaire ou ramifié, substitué ou non substitué, comportant de 1 à 15 atomes de carbone, un groupe cycloalkyle, acyle, aryle ou hétéroaryle comportant de 1 à 15 atomes de carbone, -OH,-NH₂, un halogène, -NH-alkyle, -N(alkyle)₂ ou -N⁺(alkyle)₃ ou R1 et R2 ou R2 et R3 peuvent représenter un radical de pontage cycloalkyle, cycloalcényle, aryle ou hétérocyclique, substitué ou non substitué, et
R6 et R7 représentent indépendamment l'un de l'autre un atome de carbone secondaire, tertiaire ou quaternaire et
Q représente un groupe substitué ou non substitué de pontage de R6 et R7 comprenant de 1 à 10 atomes de carbone,
**caractérisé en ce que** l'on fait réagir
a) une cétone de la formule générale (II) 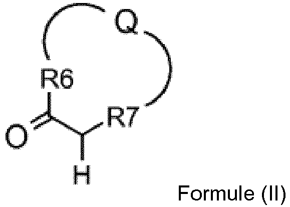 dans laquelle les radicaux R6, R7 et Q sont identiques à la formule générale (I), en présence d'un phosphonate de la formule générale (III) 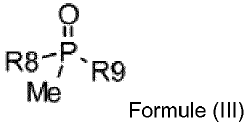 dans laquelle R8 et R9 représentent chacun indépendamment l'un de l'autre un groupe alcoxy linéaire ou ramifié, substitué ou non substitué, comportant de 1 à 15 atomes de carbone,
avec un benzonitrile de la formule générale (IV) 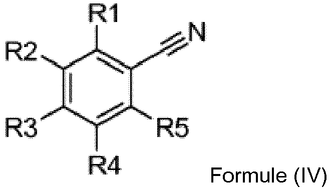 dans laquelle les radicaux R1, R2, R3, R4 et R5 sont identiques à la formule générale (I), puis
b) la cétone alpha, bêta-insaturée, obtenue à l'étape a), de la formule générale (V) 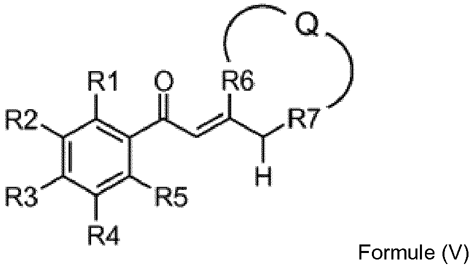 dans laquelle les radicaux R1 à R7 et Q sont identiques à la formule générale (I), est hydrogénée sélectivement pour donner une cétone de formule générale (I).

2. Procédé selon la revendication 1, **caractérisé en ce que** la cétone de la formule générale (II) comporte au moins une double liaison semi-cyclique ou exocyclique.

3. Procédé selon l'une des revendications 1 ou 2, **caractérisé en ce que** la partie de pontage - CH-R7-Q-R6- de la cétone de la formule générale (II) est un hydrocarbure.

4. Procédé selon l'une des revendications 1 à 3, **caractérisé en ce que** la cétone de la formule générale (II) est une dihydrocarvone.

5. Procédé selon l'une des revendications 1 à 4, **caractérisé en ce que** les radicaux R8 et R9 du phosphonate de la formule générale (III) représentent chacun indépendamment les uns des autres des radicaux méthoxy, éthoxy, propoxy, butoxy ou isopropoxy.

6. Procédé selon l'une des revendications 1 à 5, **caractérisé en ce que** les radicaux R1 à R5 représentent chacun indépendamment les uns des autres l'hydrogène, des groupes méthyle ou des groupes méthoxy, les groupes méthoxy étant particulièrement préférés.

7. Procédé selon l'une des revendications 1 à 6, **caractérisé en ce que** l'agent d'hydrogénation est choisi dans le groupe des borohydrures de métaux alcalins et est plus préférentiellement le borohydrure de sodium.

8. Cétone de la formule générale (VI), 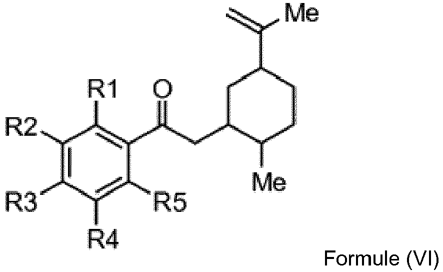
dans laquelle R1, R2, R3, R4 et R5 représentent chacun indépendamment les uns des autres l'hydrogène, -NO₂, un groupe alcoxy linéaire ou ramifié, substitué ou non substitué, comportant de 1 à 15 atomes de carbone, un groupe alkyle linéaire ou ramifié, substitué ou non substitué, comportant de 1 à 15 atomes de carbone, un groupe cycloalkyle, acyle, aryle ou hétéroaryle comportant de 1 à 15 atomes de carbone, -OH, -NH₂, un halogène,-NH-alkyle, -N(alkyle)₂ ou -N⁺(alkyle)₃ ou R1 et R2 ou R2 et R3 peuvent représenter un radical de pontage cycloalkyle, cycloalcényle, aryle ou hétérocyclique, substitué ou non substitué,
pouvant être produite par
a) la réaction de la dihydrocarvone avec un benzonitrile de la formule générale (IV) en présence d'un phosphonate de la formule générale (III), puis
b) par hydrogénation sélective de la cétone alpha, bêta-insaturée, obtenue à l'étape a), pour donner une cétone de la formule générale (VI).

9. Agent contenant une cétone de la formule générale (I) 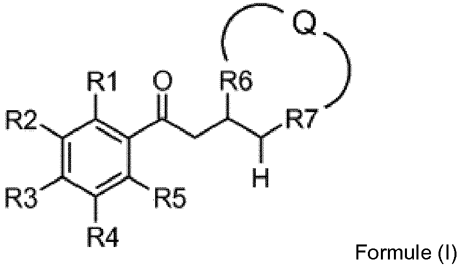
dans laquelle R1, R2, R3, R4 et R5 peuvent représenter chacun indépendamment les uns des autres, l'hydrogène, -NO₂, un groupe alcoxy linéaire ou ramifié, substitué ou non substitué, comportant de 1 à 15 atomes de carbone, un groupe alkyle linéaire ou ramifié, substitué ou non substitué, comportant de 1 à 15 atomes de carbone, un groupe cycloalkyle, acyle, aryle ou hétéroaryle comportant de 1 à 15 atomes de carbone, -OH,-NH₂, un halogène, -NH-alkyle, -N(alkyle)₂ ou -N⁺(alkyle)₃ ou R1 et R2 ou R2 et R3 peuvent représenter un radical de pontage cycloalkyle, cycloalcényle, aryle ou hétérocyclique, substitué ou non substitué, et
R6 et R7 représentent indépendamment l'un de l'autre un atome de carbone secondaire, tertiaire ou quaternaire et
Q représente un groupe substitué ou non substitué de pontage de R6 et R7 comprenant de 1 à 10 atomes de carbone,
pouvant être produit par un procédé, **caractérisé en ce que** l'on fait réagir
a) une cétone de la formule générale (II) 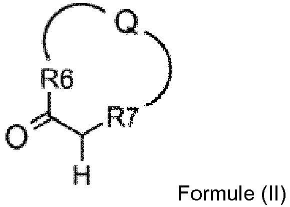 dans laquelle les radicaux R6, R7 et Q sont identiques à la formule générale (I), en présence d'un phosphonate de la formule générale (III) 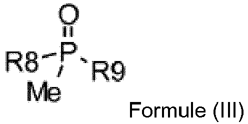 dans laquelle R8 et R9 représentent chacun indépendamment l'un de l'autre un groupe alcoxy linéaire ou ramifié, substitué ou non substitué, comportant de 1 à 15 atomes de carbone,
avec un benzonitrile de la formule générale (IV) 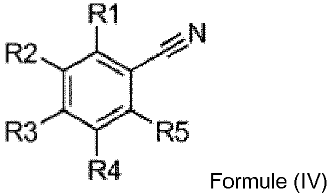 dans laquelle les radicaux R1, R2, R3, R4 et R5 sont identiques à la formule générale (I), puis
b) la cétone alpha, bêta-insaturée, obtenue à l'étape a), de la formule générale (V) 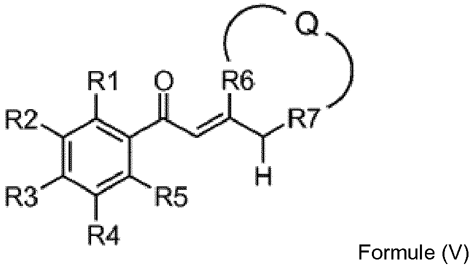 dans laquelle les radicaux R1 à R7 et Q sont identiques à la formule générale (I), est hydrogénée sélectivement pour donner une cétone de formule générale (I),
la cétone de la formule générale (I) étant une cétone de la formule générale (VI).

10. Agent selon la revendication 9, **caractérisé en ce que** l'agent est un agent cosmétique, nettoyant, ou lavant, un adhésif ou une encre d'impression.

11. Agent selon l'une des revendications 9 à 10, **caractérisé en ce qu'**il contient la cétone de la formule générale (I) dans des quantités comprises entre 0,0001 et 10 % en poids, de préférence entre 0,0005 et 5 % en poids, plus avantageusement entre 0,001 et 3 % en poids, en particulier entre 0,005 et 2 % en poids, les quantités étant rapportées à tout l'agent.
